# EUROPEAN PATENT APPLICATION

(11) **EP 2 689 717 A1**
(43) Date of publication of application: **29.01.2014**
(21) Application number: 12797183.6
(22) Date of filing: 06.06.2012
(51) Int. Cl.: A61B 1/04

(54) **ELECTRONIC ENDOSCOPE**

(30) Priority: 06.06.2011 US 201161493712 P
(71) Applicant: Fujikura Ltd., Tokyo 135-8512 (JP)
(72) Inventor: HU Wei-Zhi, Sakura-shi Chiba 285-8550 (JP); SEGI Takeshi, Sakura-shi Chiba 285-8550 (JP); NAKATATE Kenichi, Sakura-shi Chiba 285-8550 (JP); ISHIBASHI Kenichi, Sakura-shi Chiba 285-8550 (JP)
(74) Representative: Cabinet Plasseraud
(86) International application number: PCT/JP2012/064522
(87) International publication number: WO 2012/169511

(57) **Abstract**

An electronic endoscope is provided that is capable of not only the insertion portion being freely detachable, but the diameter of the distal end portion of the insertion portion being reduced so that the burden on the patient is alleviated. For that reason, the electronic endoscope of the present invention includes an elongated insertion portion inserted into a subject, a distal end of the elongated insertion portion including a solid state imaging device, an operating portion connected to an other end of the insertion portion so as to be freely attachable thereto or detached therefrom, and an interface substrate that processes RAW data obtained by the solid-state imaging device and that creates and outputs image signals, where at least part of an interface substrate is disposed inside the operating portion, and a first electrical cable that transmits the RAW data obtained by the solid-state imaging device is connected to a connector located on the interface substrate or located on the way toward the interface substrate, and when the insertion portion is being attached to or detached from the operating portion, the first electrical cable is attached or detached at a connector portion.

## Description

### TECHNICAL FIELD

The present invention relates to an electronic endoscope that utilizes a solid state imaging device, and, in particular, to an electronic endoscope in which an operating portion and an insertion portion which is inserted into a subject are mutually detachable.

Priority is claimed on U.S. Provisional Patent Application No. 61/493,712, filed June 6, 2011, the content of which is incorporated herein by reference.

### BACKGROUND ART

In order to observe the state of the interior of a body of a human or animal, or the interior of various machines and equipment, electronic endoscopes are widely used. Electronic endoscopes that are mainly used are those of a type involve inserting an image fiber in the interior of an observation subject such as a living body and optically drawing out the image of the observation subject region to the outside of the observation subject to be observed (fiberscope type), and electronic ones that involve inserting a small solid state imaging element in the interior of an observation subject, converting the image of the observation subject region into an electric signal, drawing out that electric signal to the outside of the observation subject, and observing the image with an external monitor.

The insertion portion that is inserted into an observation subject of an endoscope which utilizes an image sensor which is formed by this type of solid-state imaging device becomes contaminated by blood and the like of a patient when it is put to use. Normally, the contaminated insertion portion is used again after first being washed and sterilized, however, in recent years, in order to reduce the time and labor required by this washing and sterilizing, electronic endoscopes have been proposed in which the insertion portion is detachable and can be disposed of (see, for example, Patent Document 1).

However, in this type of electronic endoscope, because it is difficult to reduce the size of the external shape of the substrate on which the solid-state imaging device is mounted, it has been difficult to reduce the diameter of the distal end portion of the insertion portion. As a consequence, the diameter of the distal end portion of the insertion portion ends up becoming larger and this has posed a considerable burden on patients.

### PRIOR ART DOCUMENTS

### PATENT DOCUMENTS

[Patent Document 1] Japanese Unexamined Patent Application, First Publication No. H06-254047

### DISCLOSURE OF INVENTION

### PROBLEMS TO BE SOLVED BY THE INVENTION

The present invention was made in view of the above described circumstances, and it is an object thereof to provide an electronic endoscope in which not only is the insertion portion freely detachable, but the diameter of the distal end portion of the insertion portion is reduced so that the burden on the patient is alleviated.

### MEANS FOR SOLVING THE PROBLEMS

Accordingly, the electronic endoscope of the present invention includes: an elongated insertion portion that is inserted into a subject, a distal end of the elongated insertion portion including a solid state imaging device; an operating portion that is connected to an other end of the insertion portion so as to be freely attachable thereto or detached therefrom; and at least part of an interface substrate that creates and outputs image signals from RAW data obtained by the solid-state imaging device is located inside the operating portion. A first electrical cable that transmits the RAW data obtained by the solid-state imaging device is connected to a connector located on the interface substrate or located on the way toward the interface substrate, and when the insertion portion is being attached to or detached from the operating portion, the first electrical cable is attached or detached at a connector portion.

According to the above-described structure, by executing the conversion of the RAW data at a circuit substrate that is placed in the operating portion, it is possible to keep the electrical circuit mounted on a solid-state imaging device to a minimum. As a result, the diameter of the distal end of the insertion portion can be reduced. Moreover, because it is no longer necessary for the circuit substrate to be located in the insertion portion, the insertion portion can be manufactured at a lower cost, and in a form that is extremely suitable for a disposable insertion portion.

Furthermore, because the electrical cables are electrically connected together at the same time as the insertion portion is mounted on the operating portion, the mounting of the insertion portion on the operating portion can be performed more easily. In addition, by making the electrical cables mutually detachable, it is possible to increase the degree of freedom in the placement of the circuit substrate.

In the above-described electronic endoscope, it is preferable for an optical fiber to be incorporated inside the insertion portion, and for the optical fiber to transmit light from a light source which is provided at the other end of the insertion portion such that the light is emitted from the distal end of the insertion portion.

According to the above-described structure, when making an observation, the observation can be made while light is being irradiated in front of the distal end of the insertion portion.

In the above-described electronic endoscope, it is preferable for a light-emitting element to be provided inside the distal end of the insertion portion, for power to be supplied to the light-emitting element from the interface substrate by means of a second electrical cable, for the second electrical cable to be connected to a connector located on the interface substrate or located on the way toward the interface substrate, and when the insertion portion is being attached to or detached from the operating portion, for the first electrical cable to be attached or detached at a connector portion.

According to the above-described structure, because a new light source is not necessary in order for the light-emitting element to emit light, the structure can be simplified. In addition, by making the electrical cables mutually detachable, it is possible to increase the degree of freedom in the placement of the circuit substrate.

In the electronic endoscope, the interface substrate may be divided into a first substrate portion that is mounted with an analog/digital conversion circuit converting the RAW data obtained by the solid-state imaging device into a digital data signal and a second substrate portion that is mounted with an image signal creating circuit processing the digital data signal and creating an image signal. In this case, it is preferable that the first substrate portion is disposed inside the insertion portion and the second substrate portion be disposed inside the operating portion.

According to the above-mentioned structure, since a signal passing through a portion connected to a connector is digitalized already, it is possible to reduce as much as possible the adverse influence due to collapse or attenuation of a signal waveform in a connecting portion using a connector. As a result, it is possible for the length of the electrical cable (the length of a transmission line) to be relatively large.

As described above, when the interface substrate is divided into the first substrate portion and the second substrate portion, both the first substrate portion and the second substrate portion may be disposed inside the operating portion. In this case, it is preferable that the first substrate portion and the second substrate portion overlap with a gap therebetween so that plate faces of the first and the second portions are parallel to each other and that the first substrate portion and the second substrate portion be connected to each other.

In the electronic endoscope, the first substrate portion may be disposed inside the insertion portion, the second substrate portion may be disposed inside the operating portion, and the first substrate portion and the second substrate portion may be connected to each other via a connector.

According to the above-mentioned structure, it is possible to make the overall interface substrate including the first substrate portion and the second substrate portion more compact and thus it is possible to reduce the size of the operating portion receiving the interface substrate.

### BRIEF DESCRIPTION OF THE DRAWINGS

FIG. 1 is a side view of an electronic endoscope according to an embodiment of the present invention.
FIG. 2 is a bottom view of the electronic endoscope according to an embodiment of the present invention.
FIG. 3 is an exploded side view of the electronic endoscope according to an embodiment of the present invention.
FIG. 4A is an enlarged cross-sectional view taken along a line A-A shown in FIG. 1.
FIG. 4B is a view looking in the direction of an arrow B shown in FIG. 1.
FIG. 5A is a view showing an imaging module.
FIG. 5B is a view showing a light guide.
FIG. 5C is a view showing the imaging module and the light guide in combination.
FIG. 6 is an enlarged side view of a distal end unit.
FIG. 7 is an enlarged view of a connecting portion that connects an insertion portion and an operating portion.
FIG. 8 is a schematic view showing an example of an electrical connection structure of an electronic endoscope.
FIG. 9 is a block diagram illustrating an example of the circuit structure of an interface substrate.
FIG. 10 is a block diagram illustrating an example of the circuit structure when the interface substrate is divided into two portions.
FIG. 11 is a schematic view illustrating an example of an electrical connection structure of the electronic endoscope when the interface substrate is divided into two portions.
FIG. 12 is an enlarged view illustrating an example of the connecting portion that connects the insertion portion and the operating portion when the interface substrate is divided into two portions.
FIG. 13 is a block diagram illustrating another example of the circuit structure when the interface substrate is divided into two portions.
FIG. 14 is an enlarged view illustrating another example of the connecting portion that connects the insertion portion and the operating portion when the interface substrate is divided into two portions.
FIG. 15 is an enlarged view illustrating one example of a substrate dividing structure and the structure of the connecting portion that connects the insertion portion and the operating portion when the interface substrate is divided into two portions.
FIG. 16 is an enlarged view illustrating another example of the substrate dividing structure and the structure of the connecting portion that connects the insertion portion and the operating portion when the interface substrate is divided into two portions.
FIG. 17 is an enlarged view of other example of the connecting portion that connects the insertion portion and the operating portion when the interface substrate is divided into two portions.
FIG. 18A is a cross-sectional view showing an insertion portion body of another embodiment.
FIG. 18B is a cross-sectional view showing an insertion portion body of another embodiment.

### EMBODIMENTS FOR CARRYING OUT THE INVENTION

Embodiments of the present invention are described in detail with reference to the drawings.

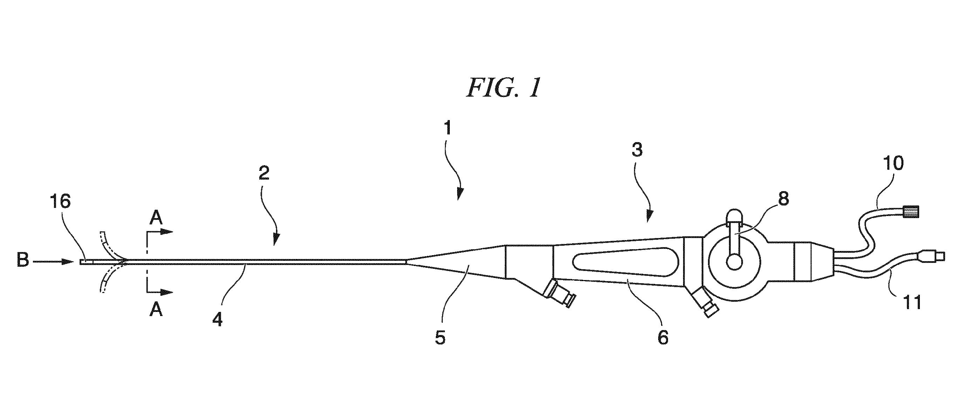

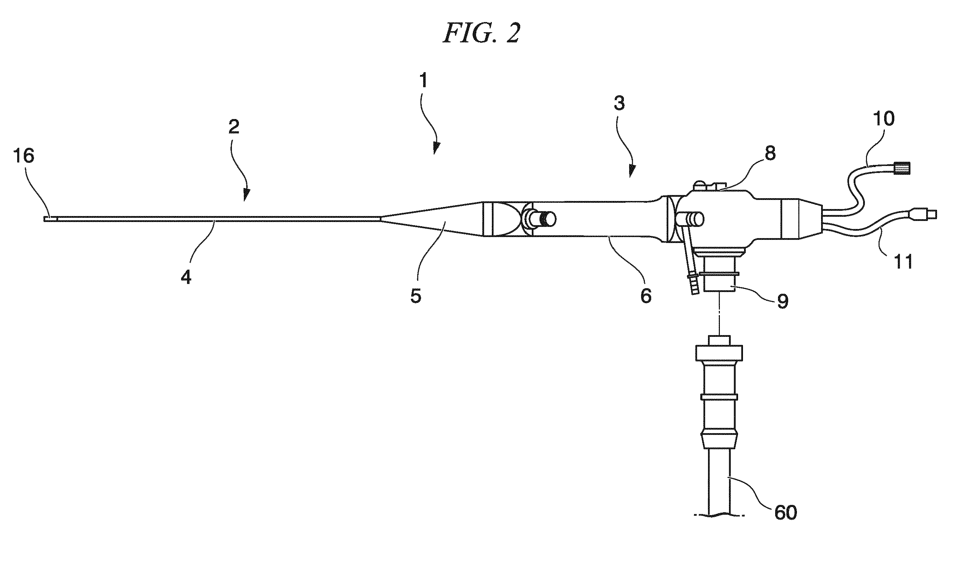

FIG. 1 is a side view showing the overall structure of an electronic endoscope 1 of this embodiment, while FIG. 2 is a bottom view thereof.

As is shown in FIG. 1 and FIG. 2, the electronic endoscope 1 is formed by an elongated insertion portion 2 that is inserted into an location to be observed inside a subject, and by an operating portion 3 that is provided at the other end of this insertion portion, and that is used to grip and operate the electronic endoscope 1.

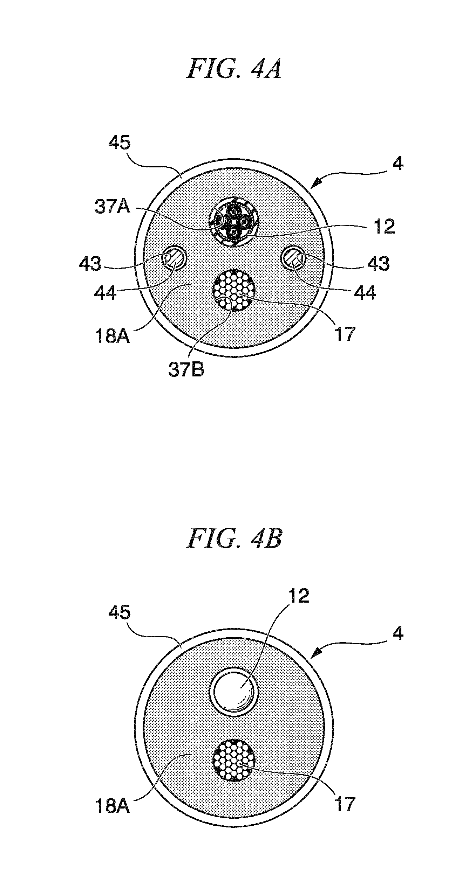

The insertion portion 2 is formed by an insertion portion main body 4 and a rigid portion 5. The operating portion 3 has an operating portion main body 6, an operating lever 8 that is used to perform a bending operation on the vicinity of the distal end of the insertion portion 2, a light guide connecting portion 9, and an analog image cable 10 and digital image cable 11 that are used to output acquired images to the outside. A light guide 17 (described below: see FIG. 4) that is placed inside the insertion portion 2 is constructed such that it is able to be connected to an external light guide 60 via the light guide connecting portion 9, and light transmitted through the external light guide 60 is introduced into the electronic endoscope 1 as a result of the external light guide 60 being connected to the light guide 17. Moreover, as is described below, by operating the handle 8 so as to drive an angle wire 44, a bending operation can be performed on the vicinity of the distal end of the insertion portion 2.

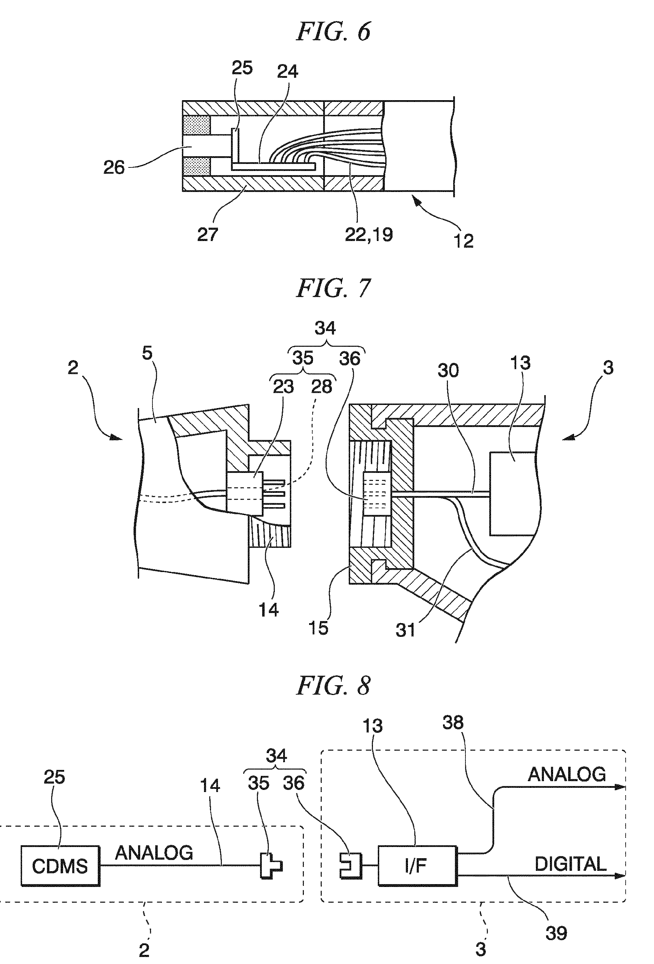

The electronic endoscope 1 observes images of a subject that are photographed by a solid-state imaging device 25 (see FIG. 6) via an objective lens system 26 (see FIG. 6) which is provided in a distal end portion 16 of the insertion portion 2.

The structure of the electronic endoscope 1 of the present embodiment will now be described in detail.

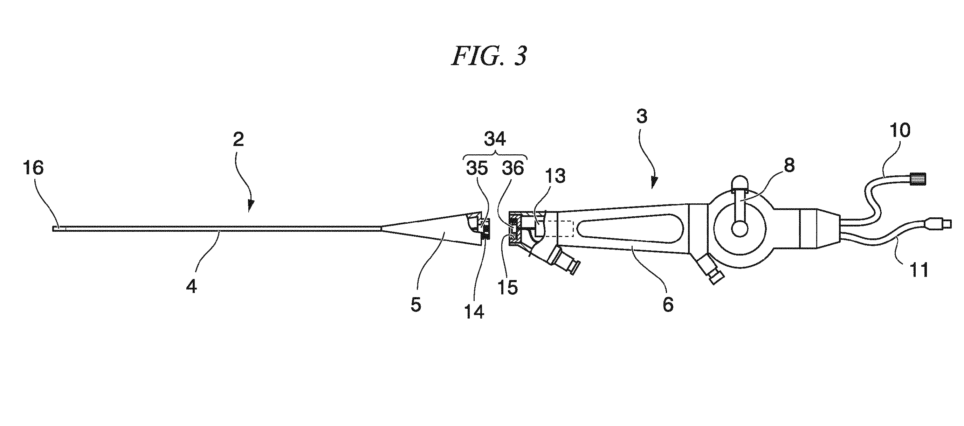

As is shown in FIG. 3, in the electronic endoscope 1 of the present embodiment the insertion portion 2 and the operating portion 3 are both mutually detachable. Specifically, they are connected such that they can be freely attached to each other and also detached from each other by means of a male connecting portion 14 which is provided in the rigid portion 5 of the insertion portion 2 and a female connecting portion 15 which is provided in the operating portion main body 6. An interface substrate 13 is also integrally fixed inside the operating portion main body 6.

The insertion portion 2 and the operating portion 3 are electrically connected to each other via a connector 34. That is, the connector 34 includes a male connector unit 35 which is provided in the male connecting portion 14 and a female connector unit 36 which is provided in the female connecting portion 15, the connector units 35 and 36 are electrically connected together, and RAW data obtained by the solid-state imaging device 25 which is provided in the distal end portion 16 of the insertion portion 2 can be transmitted to the interface substrate 13. The RAW data transmitted from the solid-state imaging device 25 is processed by a circuit on the interface substrate 13.

The transmission distance of the analog RAW data transmitted from the solid-state imaging device 25 is comparatively short (for example, 2 to 3 m), however, by first digitalizing the analog RAW data using the interface substrate 13 inside the operating portion main body 6 and then re-sending it as an image signal, the transmission distance can be lengthened.

FIG. 4A is an enlarged cross-sectional view of the insertion portion main body 4.

As is shown in FIG. 4A, a tube 18A in which a first hollow path (i.e., lumen) 37A, a second hollow path 37B, and a pair of wire holes 43 are formed extending in the longitudinal direction thereof is provided in the pole-shaped insertion portion main body 4. An imaging module 12 that is inserted through the first hollow path 37A, and the light guide 17 that is inserted through the second hollow path 37B are provided in the insertion portion main body 4. A pair of angle wires 44 that are inserted through the pair of wire holes 43, and an outer sheath 45 that covers the outermost circumference of the tube 18A and extends over the entire length thereof are provided in the insertion portion main body 4.

The angle wires 44 are wires that are used to perform a bending operation in the vicinity of the distal end of the insertion portion 2. In order to bend the vicinity portion of the distal end of the insertion portion 2, the operating lever 8 is swung in either direction from the neutral state shown in FIG. 1, so that one of the two angle wires 44 is drawn towards the lever 8, while the other angle wire 44 is pushed away from the lever 8.

A flexible resin, for example, a polyacetal resin or a polyimide resin can be preferably employed for the tube 18A. The outer sheath 45 has a layered structure formed, for example, from SUS spiral, braid, or a sheath (i.e., made from polyurethane resin or the like).

FIG. 4B shows the distal end of the insertion portion 2.

As is shown in FIG. 4B, the objective lens system 26 and the light guide 17 are exposed at the distal end of the insertion portion 2.

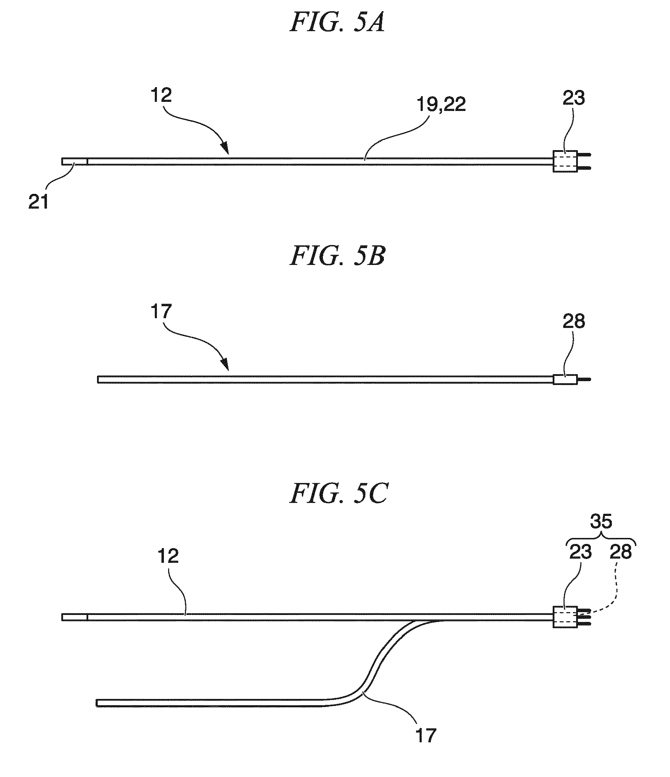

FIG. 5 shows the imaging module 12 and the light guide 17 that are housed inside the insertion portion main body 4.

As is shown in FIG. 5A, the imaging module 12 has a distal end unit 21 that is provided with the objective lens system 26 (see FIG. 6) and the solid-state imaging device 25 (see FIG. 6) and the like, a multi-coaxial cable 19 (i.e., a first electrical cable) that is formed by four coaxial cables 22 (i.e., electrical cables), and a male connector 23. The multi-coaxial cable 19 is formed, for example, by a four-core multi-coaxial cable in which the four coaxial cables 22 are bundled together. The entire bundled body formed by the four coaxial cables 22 is enclosed by a ground conductor layer (i.e., a shielding layer), and this ground conductor layer is further enclosed by a protective coating layer (i.e., a jacket). Each coaxial cable 22 is formed, for example, by a center conductor, an insulating body that surrounds the center conductor, an external conductor that surrounds the insulating body, and a protective coating layer that surrounds the external conductor.

As is shown in FIG. 5B, the distal end side of the light guide 17 is open so that light can be emitted therefrom. A male optical connector 28 is provided on the other side of the light guide 17. The light guide 17 is formed, for example, by bunching together a plurality of plastic optical fibers into a bundle.

As is shown in FIG. 5C, a male connector unit 35 can be formed by integrating the male connector 23 and the male optical connector 28 into a single unit.

FIG. 6 is an enlarged side view showing the distal end unit 21 that is provided at the distal end of the imaging module 12. As is shown in FIG. 6, the distal end unit 21 has a circular cylinder-shaped distal end sleeve 27 that forms the external shape of the distal end unit 21, a circuit substrate 24 that is provided inside the distal end sleeve 27, the solid-state imaging device 25 that is mounted in the vicinity of the distal end portion of the circuit substrate 24, and the objective lens system 26. The distal end unit 21 is constructed such that the solid-state imaging device 25 receives emission light from the objective lens system 26, and photoelectrically converts an image of the observation subject.

The distal end sleeve 27 is formed from a hard resin or from a metal such as stainless steel. The outer diameter of the distal end sleeve 27 is, for example, 1.5 mm or less, and, more preferably, is 1.0 mm or less. The distal end sleeve 27 protects the solid-state imaging device 25 and the like, and by filling the inside of the distal end sleeve 27 with an adhesive resin, the circuit substrate 24 and the objective lens system 26 and the like are fixed in position. The coaxial cables 22 that are used to transmit RAW data which has been output from the solid-state imaging device 25 are connected to the circuit substrate 24. A CMOS image sensor is employed for the solid-state imaging device 25. Positive output (AOP) and negative output (AON) of the RAW data of the solid-state imaging device 25 are output from the circuit substrate 24 by means of the coaxial cables 22. In addition, clock signals (CLK) and synchronization signals (HSYNC) are also transmitted back and forth with the interface substrate 13 by means of the coaxial cables 22. Power is also supplied by means of the coaxial cables 22.

FIG. 7 is an enlarged view of a connecting portion connecting the insertion portion 2 and the operating portion 3. As is shown in FIG. 7, the circular cylinder-shaped male connecting portion 14 is provided at the rear end of the rigid portion 5. A male thread is formed on the outer circumference of the male connecting portion 14, and is made such that it is able to connect to a female thread of the female connecting portion 15 (described below). The male connector unit 35 that is formed by the male connector 23 of the imaging module 12 and by the male optical connector 28 of the light guide 17 is fixed to the inside of the rigid portion 5.

Note that the mechanism used to connect the insertion portion 2 and the operating portion 3 is not limited to a threaded mechanism; however, it is preferable that the connections can be made without any special tools or the like having to be used. It is also possible to reverse the male-female sides of the connecting portions and the connectors.

The female connecting portion 15 is provided on the front end of the operating portion 3 such that it is able to rotate freely. A female thread is formed on the internal circumference of the female connecting portion 15, and is made such that it is able to connect to the male connecting portion 14 of the insertion portion 2. In addition, the female connector unit 36 that connects to the male connector unit 35 is fitted inside the female connecting portion 15. The female connector unit 36 is provided with an electrical connecting portion that connects to the male connector 23, and with an optical connecting portion that connects to the male optical connector 28. The female connector unit 36 is electrically connected to the interface substrate 13 via a multi-coaxial cable 30 (i.e., a first electrical cable), and is also optically connected to a light guide 31. In the same way as the multi-coaxial cable 19, the multi-coaxial cable 30 is formed, for example, by a four-core multi-coaxial cable in which four coaxial cables 22 are bundled together.

As is described above, as a result of the male connecting portion 14 being provided at the rear end of the insertion portion 2, and the female connecting portion 15 being provided at the front end of the operating portion 3, the insertion portion 2 and the operating portion 3 are detachably connected to each other. Moreover, the male connector unit 35 fixed to the male connecting portion 14 and the female connector unit 36 fixed to the female connecting portion 15, which constitute the connector 34, are electrically and optically connected to each other by connecting the male connecting portion 14 and the female connecting portion 15.

In other words, the first electrical cables (i.e., the multi-coaxial cable 19 and the multi-coaxial cable 30) are connected to connectors at points on the way toward the interface substrate 13 via the connector 34, and when the insertion portion 2 is being attached to or detached from the operating portion 3, this attachment or detachment occurs in the portion formed by the male connector unit 35 and the female connector unit 36.

Note that in this example, the first electrical cables (i.e., the multi-coaxial cable 19 and the multi-coaxial cable 30) are connected to connectors at on the way toward the interface substrate 13; however, the present invention is not limited to this. In particular, it is also possible, for example, for the first electrical cables (i.e., the multi-coaxial cable 19 and the multi-coaxial cable 30) to be connected to connectors at the interface substrate 13. In an example such as this, for example, the female connector unit 36 is mounted directly on the interface substrate 13.

FIG. 8 shows the electrical connection structure of the electronic endoscope 1 of the present embodiment. As is shown in FIG. 8, RAW data is output from the solid-state imaging device 25 of the insertion portion 2, and is input into the interface substrate 13 via the multi-coaxial cable 19 and the male connector unit 35 and the female connector unit 36 which constitute the connector 34. The interface substrate 13 serves to process the RAW data obtained by the solid-state imaging device 25 and to create and output an image signal. That is, the RAW data that is input into the interface substrate 13 is converted into an analog image signal 38 and a digital image signal 39. The analog image signal 38, for example, is directly connected to a monitor. The digital image signal 39, for example, is connected to a personal computer. Note that image signals created by the interface substrate 13 are not limited to both the analog image signal 38 and the digital image signal 39, and it is also possible for only one of the analog image signal 38 and the digital image signal 39 to be created.

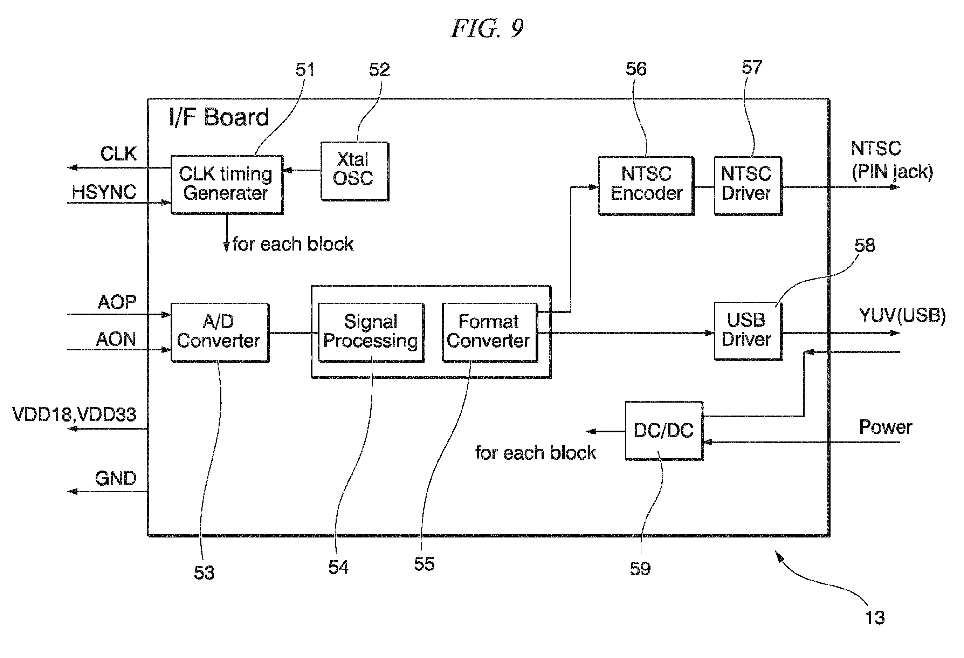

FIG. 9 is a structural view of the interface substrate 13. As is shown in FIG. 9, the interface substrate 13 has a clock synchronization circuit that is formed by a clock generating circuit 51 and a quartz crystal oscillator 52, an analog/digital conversion circuit 53, a signal processor 54, an image format converter 55, an NTSC encoding circuit 56, an NTSC driver circuit 57, a USB driver circuit 58, and a power supply circuit 59.

Hereinafter, the image processing procedure will be described.

Differential signals obtained from positive outputs AOP and negative outputs AON in the RAW data output from the solid-state imaging device 25 are digitally converted by the analog/digital conversion circuit 53. The digitalized differential signals are processed by the signal processor 54 and, as a result, a digital image signal is created. This digital image signal is then converted into a digital image signal such as YUV by the image format converter 55.

The digital image signal obtained from the image format converter 55 is then converted by the NTSC encoding circuit 56 into a composite image signal that conforms to NTSC, and this image signal is then output by the NTSC driver circuit 57 to a monitor (video display device) not shown. The digital image signal from the image format converter 55 is output by the USB driver circuit 58, for example, to a personal computer not shown.

According to the electronic endoscope 1 of the above-described embodiment, by not locating an interface circuit that digitalizes RAW data and creates an image signal in the insertion portion 2, but instead locating it in the operating portion 3 and thereby ensuring that the insertion portion 2 can be provided with a compact structure, it is possible to alleviate the burden on a patient. Moreover, because it is not necessary to locate the interface circuit that digitalizes RAW data and creates an image signal in the insertion portion 2, the insertion portion 2 can be manufactured at a lower cost which is a great advantage for a disposable insertion portion.

Moreover, because the light guide 17 that is used to irradiate light in front of the distal end portion 16 is built into the insertion portion 2, when making an observation, the observation can be made while light is being irradiated in front of the distal end portion 16 of the insertion portion 2.

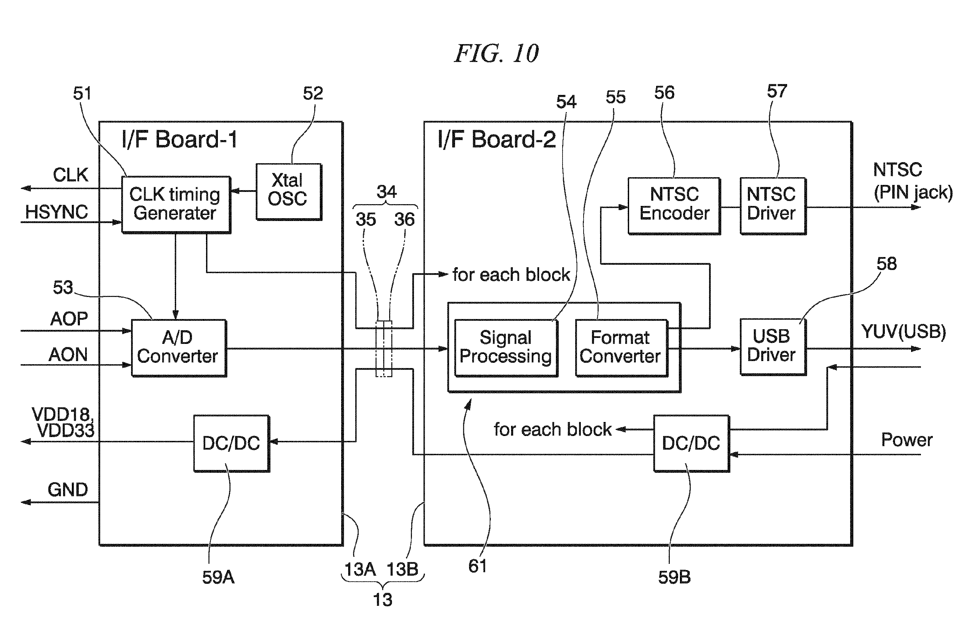

In the above-mentioned embodiment, the interface substrate 13 is formed of a single substrate and is disposed inside the operating portion 3. However, in some cases, the interface substrate 13 may be divided into two substrate portions (a first substrate portion 13A and a second substrate portion 13B as described later) depending on the functions of the circuits. Specifically, the interface substrate 13 can be divided into the first substrate portion 13A that is mounted with an analog/digital conversion circuit 53 converting the RAW data obtained by the solid-state imaging device 25 into a digital data signal and the second substrate portion 13B that is mounted with an image signal creating circuit 61 processing the digital data signal and creating an image signal. An example of the specific circuit structure in which the interface substrate 13 is divided into the first substrate portion 13A and the second substrate portion 13B in this way is shown in FIG. 10.

In the example shown in FIG. 10, the overall circuit structure including the first substrate portion 13A and the second substrate portion 13B is substantially the same as the interface substrate 13 shown in FIG. 9, but the overall circuit structure is divided into two portions, one portion (a portion including the analog/digital conversion circuit 53) of which is mounted on the first substrate portion 13A and the other portion (a portion including the image signal creating circuit 61) is mounted on the second substrate portion 13B.

Specifically, the analog/digital conversion circuit 53 converting the RAW data (analog signal) output from the solid-state imaging device 25 into a digital data signal, a clock synchronization circuit including a clock generating circuit 51 and a quartz crystal oscillator 52, and a first power supply circuit (DC/DC converter; DC voltage conversion circuit) 59A supplying a source voltage to the solid-state imaging device 25 are mounted on the first substrate portion 13A.

On the other hand, a signal processing device 54, an image format converter 55, an NTSC encoding circuit 56, an NTSC driver circuit 57, and an USB driver circuit 58 are mounted as the image signal creating circuit 61 processing the digital data signal from the analog/digital conversion circuit 53 of the first substrate portion 13A and creating an image signal (for example, an NTSC signal or a digital image signal for a PC) on the second substrate portion 13B. A second power supply circuit (DC/DC converter; DC voltage conversion circuit) 59B supplying a source voltage to the image signal creating circuit 61 and the circuits of the first substrate portion 13A is also mounted thereon.

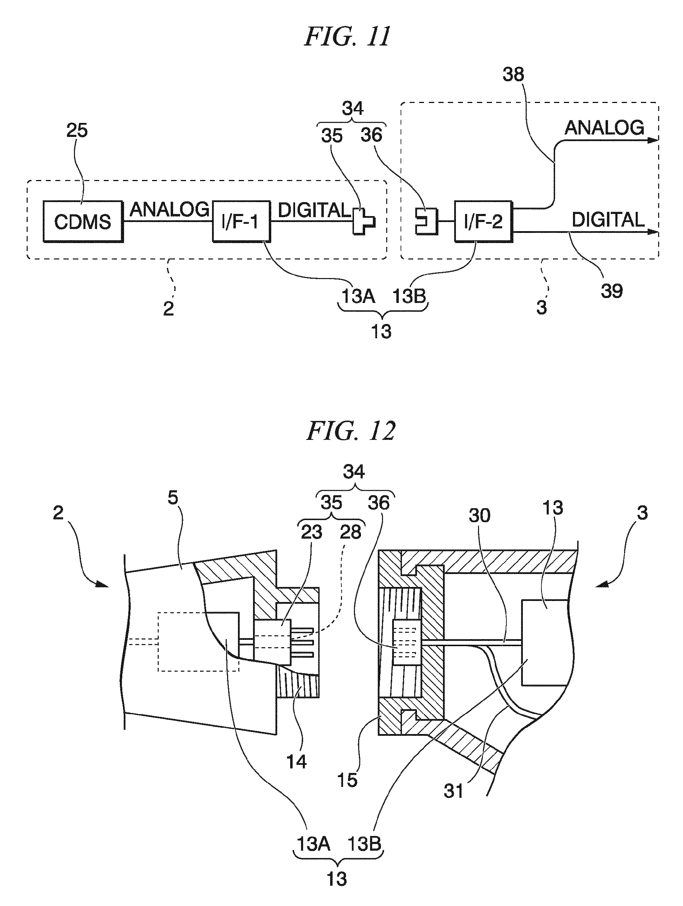

When the interface substrate 13 is divided into the first substrate portion 13A and the second substrate portion 13B in this way, as shown in FIGS. 11 and 12, the first substrate portion 13A can be disposed in the insertion portion 2 and the second substrate portion 13B can be disposed in the operating portion 3. Here, the first substrate portion 13A is typically disposed inside the rigid portion 5 of the insertion portion 2, as shown in FIG. 12.

As described above, when the first substrate portion 13A is disposed in the insertion portion 2 and the second substrate portion 13B is disposed in the operating portion 3, the first substrate portion 13A and the second substrate portion 13B are connected to each other via a connector 34. That is, the output of the first substrate portion 13A is connected to the male connector unit 35 and the input of the second substrate portion 13B is connected to the female connector unit 36.

According to this structure, a signal passing through the connecting portion using the connector 34 (the male connector unit 35 and the female connector unit 36) is a digital data signal obtained by causing the analog/digital conversion circuit 53 of the first substrate portion 13A to convert the signal in an A/D conversion manner in advance. Accordingly, it is possible to avoid an adverse influence due to the collapse or attenuation of an analog waveform on the connecting portion using the connector, which may occur when an analog signal passes through the connecting portion using the connector.

That is, since the RAW image data obtained by the solid-state imaging device 25 is an analog signal, the analog waveform may easily collapse or attenuate while transmitting the analog signal via the electrical cable. In addition, the analog waveform in the connecting portion using the connector can easily collapse or attenuate greatly when the connecting portion using the connector is interposed on the way of a transmission line. This means that it is apt not to correctly reproduce an image from the image data obtained by the solid-state imaging device 25. Accordingly, when the interface substrate 13 including the analog/digital conversion circuit 53 is not divided but the overall interface substrate is disposed in the operating portion 3 so as for the RAW image data to pass through the connecting portion using the connector, the length of the electrical cable (the length of the transmission line) should be determined in consideration of the collapse or attenuation of a waveform in the connecting portion using the connector. As a result, the length of the electrical cable (the length of the transmission line) is limited so as to be relatively small.

On the contrary, when the interface substrate 13 is divided and the first substrate portion 13A including the analog/digital conversion circuit 53 is disposed in the insertion portion 2, the signal passing through the connecting portion using the connector 34 is digitalized in advance and it is thus possible to reduce an adverse influence due to the collapse or attenuation of a signal waveform in the connecting portion using the connector as much as possible. As a result, the length of the electrical cable (the length of the transmission line) can be relatively large.

In the circuit structure shown in FIG. 10, the clock synchronization circuit including the clock generating circuit 51 and the quartz crystal oscillator 52 is mounted on the first substrate portion 13A, but the clock synchronization circuit may be mounted on the second substrate portion 13B in some cases. Here, when it is desirable to speed up the clock, it is preferable that the clock synchronization circuit be mounted on the first substrate portion 13A closer to the solid-state imaging device 25 than to the connecting portion using the connector 34.

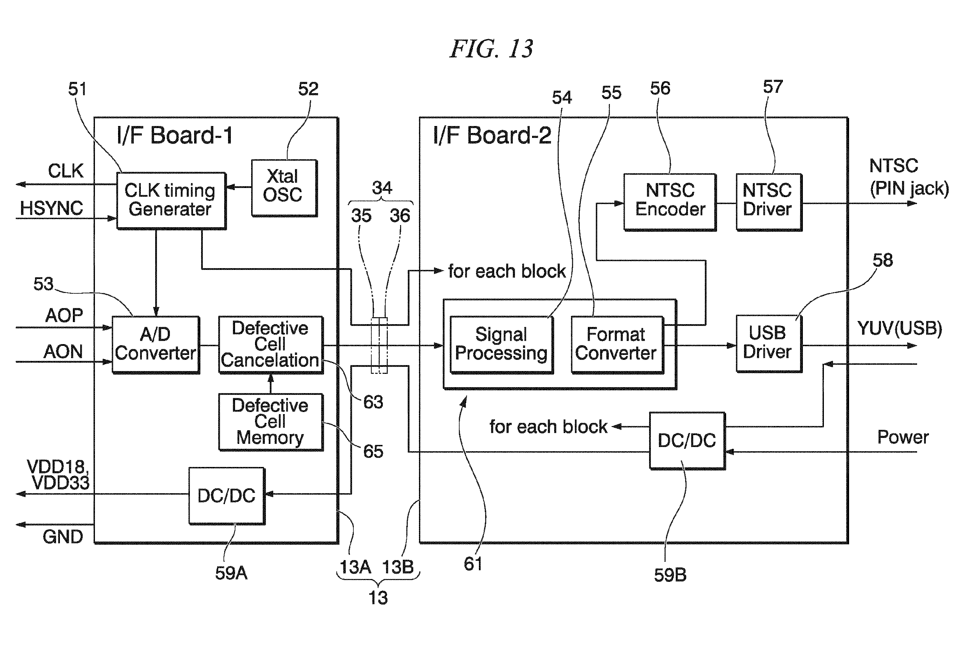

Another example of the circuit structure in which the interface substrate 13 is divided into the first substrate portion 13A and the second substrate portion 13B as described above is shown in FIG. 13.

In the example shown in FIG. 13, a defective cell memory 63 storing a defective cell (a cell which does not operate or which cannot supply a normal data signal even when it operates) in advance out of plural cells (pixels) of the solid-state imaging device 25 and a defective cell signal cancelling circuit 65 cancelling a signal of the defective cell stored in the defective cell memory 63 out of image data signals from the analog/digital conversion circuit 53 are mounted on the first substrate portion 13A. The signal (digital data signal) from which the signal from the defective cell is cancelled through the defective cell signal cancelling circuit 65 is sent to the second substrate portion 13A. The other circuit structure is the same as shown in FIG. 10.

In the circuit structure shown in FIG. 13, it is possible to prevent an image from being not correctly reproduced due to the influence of the signal from the defective cell in the solid-state imaging device 25.

The defective cell memory 63 and the defective cell signal cancelling circuit 65 may be disposed in the second substrate portion 13B. However, since the defective cell to be stored in the defective cell memory 63 attending the specific solid-state imaging device 25, it is preferable to mount the defective cell memory 63 and the defective cell signal cancelling circuit 65 on the first substrate portion 13A disposed in the insertion portion 2, as shown in FIG. 13, when the insertion portion 2 including the solid-state imaging device 25 is replaced.

That is, when the insertion portion 2 is detached from the operating portion 3 at the above-mentioned connecting portion using the connector and the insertion portion 2 is replaced with a new one, the solid-state imaging device 25 is also replaced accordingly. Therefore, when cell defect information on the solid-state imaging device 25 in the insertion portion 2 is stored in advance in the defective cell memory 63 of the first substrate portion 13A in the insertion portion 2 to be newly attached, the cell defect information on the solid-state imaging device 25 of the new insertion portion 2 can be utilized just after replacing the insertion portion.

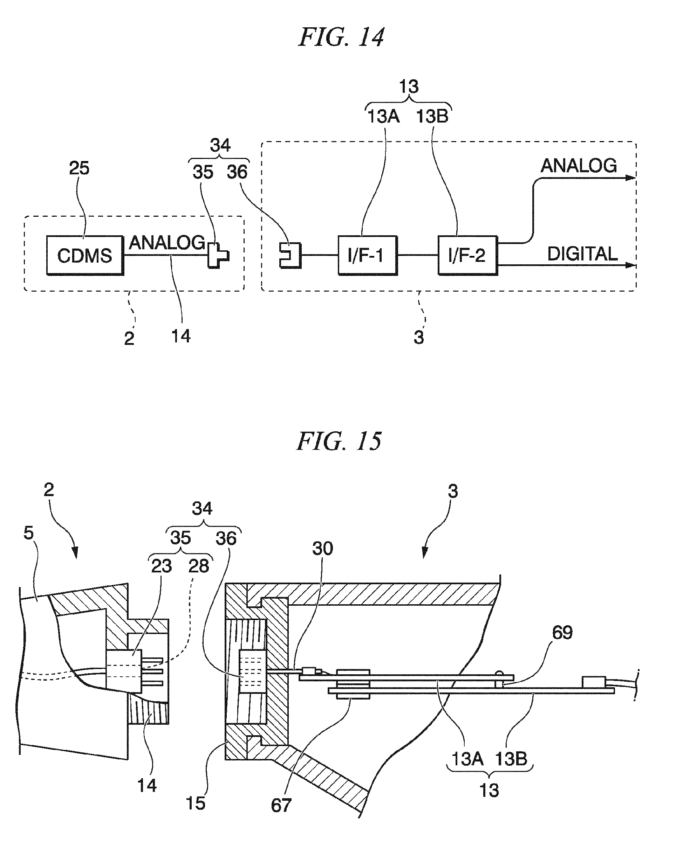

In the above description, when the interface substrate 13 is divided into two substrate portions (the first substrate portion 13A and the second substrate portion 13B), the first substrate portion 13A is disposed in the insertion portion 2 and the second substrate portion 13B is disposed in the operating portion 3. However, in some cases, both the first substrate portion 13A and the second substrate portion 13B may be disposed in the operating portion 3, as shown in FIG. 14. In this case, the cable between the first substrate portion 13A and the second substrate portion 13B can be appropriately extended depending on the shape or size of the operating portion 3 or the internal structure thereof.

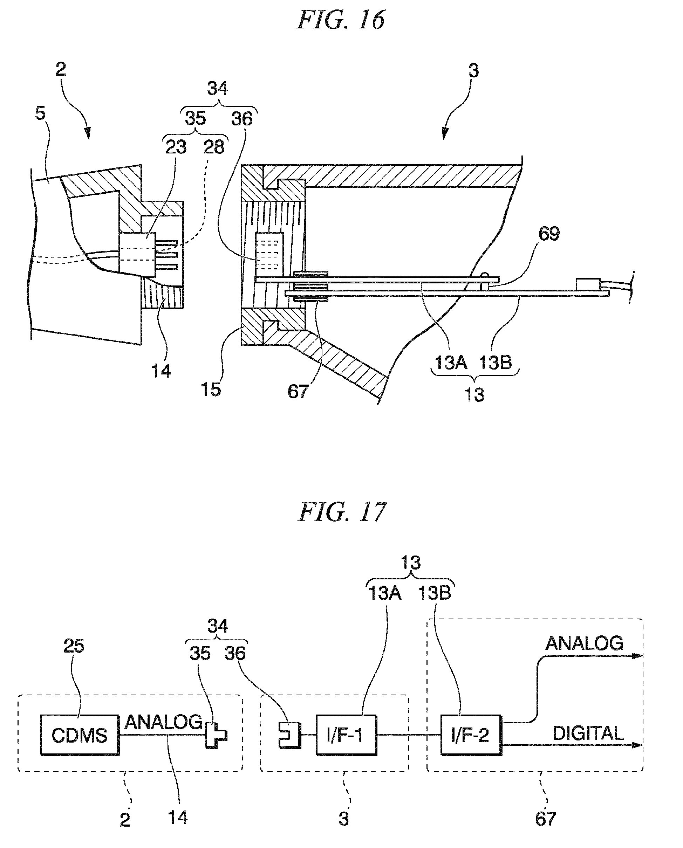

As described above, when the interface substrate 13 is divided into two substrate portions (the first substrate portion 13A and the second substrate portion 13B) and both the first substrate portion 13A and the second substrate portion 13B are disposed in the operating portion 3, it is preferable that both overlap so that the plate faces thereof are parallel to each other and both are connected to each other via a connector 67, for example, as shown in FIG. 15 or 16. A positioning and supporting pin 69 is interposed between the first substrate portion 13A and the second substrate portion 13B. The structure of the interface substrate 13 shown in FIG. 15 or 16 can be said to be a "two-layered structure". In the two-layered structure, it is possible to compact the overall interface substrate 13, compared with the case where the interface substrate 13 is not divided. Accordingly, it is possible to reduce the size of the operating portion 3 receiving the interface substrate 13.

In the example shown in FIG. 15, the female connector unit 36 constituting the side of the operating portion 3 of the connector 34 is electrically connected to the first substrate portion 13A via the multi-coaxial cable 30 (the first electrical cable). Here, it is shown that the cable 30 drawn out from the female connector unit 36 is directly connected to the first substrate portion 13A, but the cable 30 may be electrically connected to the first substrate portion 13A via a connector not shown.

In the example shown in FIG. 16, the female connector unit 36 constituting the side of the operating portion 3 of the connector 34 is directly mounted on the first substrate portion 13A. In this case, the male connector unit 35 constituting the portion of the connector 34 on the insertion portion 2 side is electrically connected directly to the female connector unit 36 attached to the first substrate portion 13A.

In the examples shown in FIGS. 15 and 16, the first substrate portion 13A and the second substrate portion 13B are detachably connected via the connector 67, but the first substrate portion 13A and the second substrate portion 13B may be fixed to each other.

When the interface substrate 13 is divided into two substrate portions (the first substrate portion 13A and the second substrate portion 13B), the first substrate portion 13A may be disposed in the operating portion 3 and the second substrate portion 13B may be disposed in a control box 67 outside the operating portion 3, as shown in FIG. 17. In this case, the control box 67 receiving the second substrate portion 13B may be disposed in the vicinity of a monitor (video image display device) or a personal computer not shown.

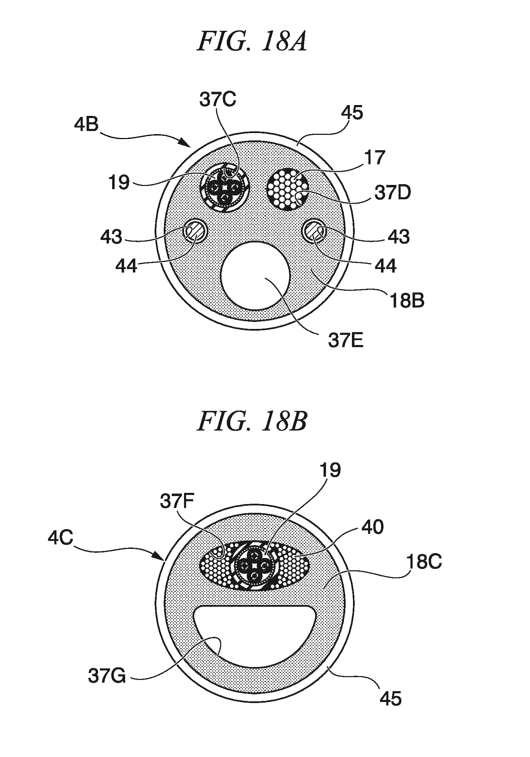

FIGS. 18A and 18B show a cross-section of an insertion portion main body of another embodiment. As is shown in FIG. 18A, in another embodiment, in addition to a hollow path 37C which is used for the multi-coaxial cable 19 and a hollow path 37D which is used for the light guide 17, an additional hollow path 37E is provided and this hollow path 37E is used as a channel. A channel can be used as a supply path for nutrient solutions or medications or the like. Note that, although omitted from the drawing, this channel has a mechanism that enables it to be attached or detached when the insertion portion 2 is attached to or detached from the operating portion 3 (described below).

Moreover, as is shown in FIG. 18B, it is also possible to employ a configuration in which two hollow paths are formed, with a light guide 40 and the multi-coaxial cable 19 being inserted through one of these hollow paths 37F, and with the other hollow path 37G being used as a channel. In the configuration shown in FIG. 18B, the wires that are used to perform a bending operation have been eliminated so that this configuration is preferably used for a catheter tract or the like instead of for an endoscope.

Preferred embodiments of the present invention have been described above; however, it is to be understood that these embodiments are merely examples within the range of technology intended by the present invention, and various additions, omissions, substitutions, and other modifications to the structure are possible insofar as they do not depart from the spirit or scope of the present invention. The present invention is not limited by the foregoing description, and is only limited by the range of the appended claims. It is to be understood that suitable modifications thereto are possible.

For example, the means employed to irradiate light in front of the distal end portion 16 is not limited to an optical fiber, and it is possible to provide inside the distal end portion 16 a semiconductor element (i.e., an LED or light emitting element) that emits light and to use this as a substitute for an optical fiber. In this case, a structure may be employed in which the power supplied to the LED is supplied from the interface substrate 13 by means of the multi-coaxial cable 19 (i.e., the second electrical cable). Moreover, the multi-coaxial cable 19 is connected to a connector located on the interface substrate 13 or located at a point on the way toward the interface substrate 13. In such a structure, power is supplied by connecting together the male connecting portion 14 and the female connecting portion 15 so that the male connector unit 35 and the female connector unit 36 are mutually connected together.

By employing an LED, there is no need to provide a light guide so that the structure can be simplified.

### INDUSTRIAL APPLICABILITY

The present invention can be applied to an electronic endoscope that utilizes a solid state imaging device, and, in particular, to an electronic endoscope in which an operating portion and an insertion portion which is inserted into a subject are mutually detachable

### DESCRIPTION OF THE REFERENCE SYMBOLS

1: Electronic endoscope
2: Insertion portion
3: Operating portion
4: Insertion portion main body
5: Rigid portion
6: Main body
8: Operating lever
12: Imaging module
13: Interface substrate
16: Distal end portion
17: Light guide
34: Connector
45: Outer sheath
60: External light guide

## Claims

1. An electronic endoscope comprising:
an elongated insertion portion that is inserted into a subject, a distal end of the elongated insertion portion comprising a solid-state imaging device;
an operating portion that is connected to an other end of the insertion portion so as to be freely attachable thereto or detached therefrom; and
an interface substrate that processes RAW data obtained by the solid-state imaging device and that creates and outputs image signals, wherein:
at least part of the interface substrate is disposed inside the operating portion;
and
a first electrical cable that transmits the RAW data obtained by the solid-state imaging device is connected to a connector located on the interface substrate or located on the way toward the interface substrate, and when the insertion portion is being attached to or detached from the operating portion, the first electrical cable is attached or detached at a connector portion.

2. The electronic endoscope according to claim 1, wherein
an optical fiber is incorporated inside the insertion portion, the optical fiber transmitting light from a light source which is provided at the other end of the insertion portion such that the light is emitted from the distal end of the insertion portion.

3. The electronic endoscope according to claim 1, wherein:
a light-emitting element is provided inside the distal end of the insertion portion;
power is supplied to the light-emitting element from the interface substrate by means of a second electrical cable;
the second electrical cable is connected to a connector located on the interface substrate or located on the way toward the interface substrate; and
when the insertion portion is being attached to or detached from the operating portion, the first electrical cable is attached or detached at a connector portion.

4. The electronic endoscope according to claim 1, wherein the interface substrate is divided into a first substrate portion that is mounted with an analog/digital conversion circuit converting the RAW data obtained by the solid-state imaging device into a digital data signal and a second substrate portion that is mounted with an image signal creating circuit processing the digital data signal and creating an image signal, and
wherein at least the second substrate portion of the first substrate portion and the second substrate portion is disposed inside the operating portion.

5. The electronic endoscope according to claim 4, wherein the first substrate portion and the second substrate portion are disposed inside the operating portion.

6. The electronic endoscope according to claim 5, wherein the first substrate portion and the second substrate portion overlap with a gap so that plate faces of the first and the second portions are parallel to each other, and are connected to each other.

7. The electronic endoscope according to claim 4, wherein the first substrate portion is disposed inside the insertion portion, the second substrate portion is disposed inside the operating portion, and the first substrate portion and the second substrate portion are connected to each other via a connector.
